# EUROPEAN PATENT SPECIFICATION

(11) **EP 3 408 366 B1**
(45) Date of publication and mention of the grant of the patent: **08.12.2021**
(21) Application number: 16825399.5
(22) Date of filing: 21.12.2016
(51) Int. Cl.: C11D 3/386, C11D 11/00

(54) **METHOD FOR CLEANING A MEDICAL OR DENTAL INSTRUMENT**
VERFAHREN ZUR REINIGUNG VON MEDIZINISCHE ODER ZAHNÄRZTLICHE INSTRUMENTEN
PROCÉDÉ DE NETTOYAGE D'INSTRUMENTS MÉDICAUX OU DENTAIRES

(30) Priority: 28.01.2016 EP 16153226
(43) Date of publication of application: 05.12.2018
(73) Proprietor: Novozymes A/S, 2880 Bagsvaerd (DK)
(72) Inventor: COQUILLAT, Julien, 78230 Le Pecq (FR)
(74) Representative: NZ EPO Representatives
(86) International application number: PCT/EP2016/082139
(87) International publication number: WO 2017/129331

(56) References cited:
- WO-A1-01/76647
- WO-A1-2010/055052
- WO-A1-2013/004635
- WO-A1-2013/004636
- WO-A2-2009/118375
- WO-A2-2014/173980
- GB-A- 2 482 164
- US-A1- 2011 061 686

### SEQUENCE LISTING

<110> Novozymes A/S
<120> Medical and dental instrument cleaning
<130> 13042-WO-PCT
<160> 13
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 275
   <212> PRT
   <213> Bacillus amyloliquefaciens
<400> 2
<210> 3
   <211> 269
   <212> PRT
   <213> Artificial
<220>
   <223> Variant of SEQ ID NO: 1
<400> 3
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Ala Pro Ala Ser Tyr Ala Asn Ala Met Ala Val Gly Ala Thr Asp Gln
                   165                 170                 175
   Asn Asn Asn Arg Ala Ser Phe Ser Gln Tyr Gly Pro Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 269
   <212> PRT
   <213> Artificial
<220>
   <223> Variant of SEQ ID NO: 1
<400> 4
   Ala Gln Ser Val Pro Trp Gly Ile Glu Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Arg Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asp Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Gly Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Ile Leu Ser Thr Trp Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Asp Thr Trp Glu
   245 250 255
   Tyr Gly Ser Gly Leu Val Asn Ala Glu Ala Ala Thr Arg
   260 265
<210> 5
   <211> 4
   <212> PRT
   <213> Artificial
<220>
   <223> Subtilisin inhibitor
<220>
   <221> MISC_FEATURE
   <222> (1)..(1)
   <223> Acetyl-Leu
<220>
   <221> MISC_FEATURE
   <222> (4)..(4)
   <223> Tyr-H
<400> 5
   Leu Gly Ala Tyr
   1
<210> 6
   <211> 4
   <212> PRT
   <213> Artificial
<220>
   <223> Subtilisin inhibitor
<220>
   <221> MISC_FEATURE
   <222> (1)..(1)
   <223> Acetyl-Phe
<220>
   <221> MISC_FEATURE
   <222> (4)..(4)
   <223> Tyr-H
<400> 6
   Phe Gly Ala Tyr
   1
<210> 7
   <211> 4
   <212> PRT
   <213> Artificial
<220>
   <223> Subtilisin inhibitor
<220>
   <221> MISC_FEATURE
   <222> (1)..(1)
   <223> Acetyl-Tyr
<220>
   <221> MISC_FEATURE
   <222> (4)..(4)
   <223> Tyr-H
<400> 7
   Tyr Gly Ala Tyr
   1
<210> 8
   <211> 4
   <212> PRT
   <213> Artificial
<220>
   <223> Subtilisin inhibitor
<220>
   <221> MISC_FEATURE
   <222> (1)..(1)
   <223> Acetyl-Phe; MeO-CO-Phe; MeSO2-Phe; or EtSO2-Phe
<220>
   <221> MISC_FEATURE
   <222> (4)..(4)
   <223> Leu-H
<400> 8
   Phe Gly Ala Leu
   1
<210> 9
   <211> 4
   <212> PRT
   <213> Artificial
<220>
   <223> Subtilisin inhibitor
<220>
   <221> MISC_FEATURE
   <222> (1)..(1)
   <223> Acetyl-Phe or MeO-CO-Phe
<220>
   <221> MISC_FEATURE
   <222> (4)..(4)
   <223> Tyr-H
<400> 9
   Phe Gly Ala Phe
   1
<210> 10
   <211> 4
   <212> PRT
   <213> Artificial
<220>
   <223> Subtilisin inhibitor
<220>
   <221> MISC_FEATURE
   <222> (1)..(1)
   <223> Acetyl-Phe
<220>
   <221> MISC_FEATURE
   <222> (4)..(4)
   <223> Tyr-H
<400> 10
   Phe Gly Val Tyr
   1
<210> 11
   <211> 4
   <212> PRT
   <213> Artificial
<220>
   <223> Subtilisin inhibitor
<220>
   <221> MISC_FEATURE
   <222> (1)..(1)
   <223> Acetyl-Phe
<220>
   <221> MISC_FEATURE
   <222> (4)..(4)
   <223> Met-H
<400> 11
   Phe Gly Ala Met
   1
<210> 12
   <211> 4
   <212> PRT
   <213> Artificial
<220>
   <223> Subtilisin inhibitor
<220>
   <221> MISC_FEATURE
   <222> (1)..(1)
   <223> Acetyl-Trp
<220>
   <221> MISC_FEATURE
   <222> (4)..(4)
   <223> Tyr-H
<400> 12
   Trp Leu Val Tyr
   1
<210> 13
   <211> 4
   <212> PRT
   <213> Artificial
<220>
   <223> Subtilisin inhibitor
<220>
   <221> MISC_FEATURE
   <222> (1)..(1)
   <223> MeO-P(OH)(O)-Leu
<220>
   <221> MISC_FEATURE
   <222> (4)..(4)
   <223> Leu-H
<400> 13
   Leu Gly Ala Leu
   1

## Description

### FIELD OF THE INVENTION

The present invention relates to methods for cleaning medical and dental instruments.

### BACKGROUND

In the health care industry, medical instruments must be thoroughly cleaned and sanitized before being reused. Cleaning processes include multiple steps where some steps may be automated and some steps may be manual. The instruments cleaned may be heavily soiled with biological soils, in particular protein based soils; and the instruments itself may be sharp, small or irregular shaped, thus impairing physical access to the soils.

WO 2014/110015 relates to compositions and methods for cleaning medical and dental instruments. The disclosed compositions are preferably non-foaming or generate low foam to allow visual inspection of the cleaning process as well as safe handling of the instruments. The disclosed compositions preferably employ select proteases, a carbonate and a nonionic surfactant.

GB 2482164 discloses medical cleaning compositions comprising enzymes, and boron and calcium stabilizing compounds.

### SUMMARY

Surprisingly, it has been discovered that the disclosed compositions and methods provide improved removal of soils on surfaces, such as medical and dental instruments. The disclosed compositions are stable and operable over a pH and temperature range.

Thus, the invention provides a method for cleaning a medical or dental instrument comprising:
a) soaking the medical or dental instrument in an aquous composition comprising
   i) from 0.001% w/w to 1% w/w of a protease, wherein the protease is a subtilisin, and
   ii) from 0.001% w/w to 1% w/w of a peptide aldehyde protease stabilizer;
b) optionally washing the instrument with a liquid detergent composition; and
c) rinsing the instrument;
wherein the peptide aldehyde protease stabilizer has the formula P-B²-B¹-B⁰-H or a hydrosulfite adduct thereof having the formula P-B²-B¹-N(H)-CHR-CHOH-SO₃M, wherein
i) H is hydrogen;
ii) B⁰ is a single amino acid residue with L- or D-configuration of the formula -NH-CH(R)-C(=O)-;
iii) B¹ and B² are independently single amino acid residues;
iv) R is independently selected from the group consisting of C₁₋₆ alkyl, C₆₋₁₀ aryl or C₇₋₁₀ arylalkyl optionally substituted with one or more, identical or different, substituents R';
v) R' is independently selected from the group consisting of halogen, -OH, -OR", -SH, -SR", - NH₂, -NHR", -NR"₂, -CO₂H, -CONH₂, -CONHR", -CONR"₂, -NHC(=N)NH₂;
vi) R" is a C₁₋₆ alkyl group;
vii) P is an N-terminal protection group; and
viii) M is H or an alkali metal, preferably Na or K.

These and other embodiments will be apparent to those skilled in the art and others in view of the following description and embodiments.

### DETAILED DESCRIPTION

The present disclosure is directed to compositions and methods that are effective at removing biological soils on medical and dental instruments. The disclosed compositions provide improved cleaning compared to known enzymatic compositions for cleaning medical and dental instruments.

The inventors have found that by including a peptide protease stabilizer in a protease containing cleaning composition for medical or dental instruments, the composition exhibits improved cleaning efficiency. Other protease stabilizers are known in the art, such as boric acid or boronic acids; however, these stabilizers do not result in improved cleaning efficiency when included in similar cleaning compositions.

Protease stabilizers are normally used to inhibit protease activity during storage of concentrated liquid protease containing products to reduce proteolysis of enzymes and other proteins. In the concentrated product the stabilizer is bound to the protease, but upon aquous dilution, the stabilizer is released from the protease. Surprisingly, the present invention demonstrates that the released peptide-derived protease stabilizer improves cleaning of medical and dental instruments when used in a protease-containing cleaning composition.

### Medical and dental instruments

The medical and dental instruments that are cleaned, washed, and/or soaked according to the invention, include medical and dental devices, instruments, or equipment, including any of the various medical or dental instruments or devices that can benefit from cleaning with the enzyme cleaning composition. Exemplary medical and dental instruments and devices include instruments, devices, tools, appliances, apparatus, and equipment used in medicine or dentistry, including those than can be cold sterilized, soaked or washed and then heat sterilized, or otherwise benefit from cleaning in the disclosed compositions. These various instruments, devices and equipment include, but are not limited to: diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment, and the like, or combinations thereof.

### Cleaning

The medical and dental instruments are cleaned, according to the invention, by soaking it in an aquous composition (cleaning composition) comprising from about 0.001% w/w to 1% w/w of a protease, and from about 0.001% w/w to 1% w/w of a peptide protease stabilizer, preferably a peptide aldehyde protease stabilizer, or a hydrosulfite adduct thereof. The aquous composition may further comprise one or more other enzymes (non-protease enzymes), and other suitable cleaning ingredients, such as surfactant(s), wetting agent(s), and/or other ingredients as described below under "liquid detergent composition". The composition may also include a source of alkalinity. The pH of the aquous composition is typically alkaline; preferably the pH is in the range from pH 7 to pH 10. pH may be measured directly in the composition or in a 5% solution in water

In the context of the present invention, "soaking" means to make the medical and dental instruments wet with the above-mentioned cleaning composition, or to immerse, or partly immerse, such instruments in the cleaning composition for a period of time, or a combination of both. A medical or dental instrument may be only partly soaked with the cleaning composition if only a part of the instrument needs cleaning. For example, it may be desirable to avoid contacting electronic circuits or other electrical parts with the aquous cleaning composition.

The term "cleaning" means reducing the amount of proteinaceous soils; thus, "soil" in the context of the invention is a protein substance degradable by a protease. In a particular embodiment, the soils of the invention are blood, blood constituents, blood proteins, fibrin, albumin and/or hemoglobin.

The medical or dental instruments are soaked in the aquous composition for a time sufficient to be effective at reducing or removing the soils on the instruments. Depending on the level of soiling, the instruments may be soaked in the aquous composition for at least 1 minute. Soaking may continue even after cleaning is complete. For example, for convenience cleaning may continue over night, or even for up to 24 hours. The upper time limit is determined by the robustness of the instruments, to avoid damaging them.

The medical or dental instruments may be soaked in the aquous composition at a temperature between room temperature and 90 degrees Celsius, preferably between 20 and 90 degrees Celsius, more preferably between 30 and 80 degrees Celsius, even more preferably between 30 and 70 degrees Celsius, and most preferably between 30 and 60 degrees Celsius.

Soaking of the medical and dental instruments may be carried out with or without mechanical action (such as shaking or stirring) in a tray, tub, pan, or sink; or by spraying such as through an instrument washer; by ultrasonic treatment, treatment in a cart or cage washer; by manually applying it with a hand-held bottle as either a spray or a foam; or by mechanized washing in a laboratory glass machine washer.

In an embodiment, the cleaning of medical or dental devices, and/or non-medical types of equipment, takes place in a (Medical) Washer-Disinfector according to EN ISO 15883-1 (or as described in "Class II Special Controls Guidance Document: Medical Washers and Medical Washer-Disinfectors; Guidance for the Medical Device Industry and FDA Review Staff", U.S. Food and Drug Administration, Feb. 2002), using the methods of the invention.

During soaking of the soiled medical or dental instruments, medical devices, or portions of medical devices are contacted with an effective amount of the aquous composition comprising the protease and the peptide aldehyde protease stabilizer. The actual amount of the composition used will be based on the judgment of user, and will depend upon factors such as the particular product formulation of the composition, the concentration of the composition, the number of soiled articles to be soaked and the degree of soiling of the articles. Subsequently, the items may be subjected to a manual or machine washing or rinsing method, involving either further washing steps and use of a detergent composition, and/or to a manual or machine rinsing method. The disclosed compositions and methods are effective at removing the soils on instruments by reducing the amount of scrubbing required in the cleaning process.

The aquous composition comprising the protease and the peptide aldehyde protease stabilizer can be employed in a variety of machines that wash or soak instruments, such as medical or dental instruments or devices. Such machines can be charged manually with liquid forms, or powder or other solid forms of the composition. Such machines can also automatically dispense the disclosed compositions. Such dispensing can include dissolving the cleaning composition to form a first liquid concentrate composition, optionally diluting the first liquid concentrate composition to yield a second liquid concentrate composition (that is less concentrated), and diluting the second liquid concentrate composition into the wash or soak chamber to form the use composition. The use composition can be used to wash or soak the instruments. Such dispensing can also include dissolving a solid cleaning composition once to form a use solution.

In addition to cleaning medical and dental instruments and devices, the disclosed aquous cleaning compositions can also be used to clean other surfaces, including food preparation equipment such as freezers, ovens, conveyors, portioners, slabbers, trimmers, slicers, hoppers, graders, scales, packaging equipment, blades, knives, meat saws, and other kitchen tools. The disclosed compositions could also be used in manual and automatic dishwashing and warewashing applications, as a pre-soak for dishes and wares, as a detergent in a powersink application, which is an open washing device with low pressure jets, and as a hard surface cleaner for use in kitchens, delis, grocery stores, butcher shops, bakeries, restaurants, and cold storage areas. It can also be used as a hard surface cleaner for glass such as that found in food retail spaces, drains, bathroom surfaces, clean-in-place equipment, tanker and delivery vehicle cleaning, and bottle washing. The disclosed compositions can be used to clean soft surfaces such as drapes, laundry, carpet, and upholstery. Finally, the disclosed compositions can be used in water treatment to help remove protein deposits in water treatment equipment and to clean membranes.

### Protease

The protease enzymes for use in the present invention are subtilisins and include those of bacterial, fungal, plant, viral or animal origin e.g. vegetable or microbial origin. Microbial origin is preferred. Chemically modified or protein engineered mutants are included.

In the context of the present invention, the subtilisin enzyme family (EC 3.4.21.62) shall be understood as described by Siezen et al., Protein Engng. 4 (1991) 719-737 and Siezen et al. Protein Science 6 (1997) 501-523. As described therein, the subtilisin family may be divided into 3 sub-groups, i.e. I-S1 ("true" subtilisins), I-S2 (highly alkaline proteases) and intracellular subtilisins.

Examples of subtilisins are those derived from *Bacillus* such as *subtilisin lentus, Bacillus lentus, subtilisin Novo, subtilisin Carlsberg, Bacillus licheniformis, subtilisin BPN', subtilisin 309, subtilisin 147* and *subtilisin* 168 described in WO 89/06279 and protease PD138 (WO 93/18140). Additional examples are described in WO 98/020115, WO 01/44452, WO 01/58275, WO 01/58276, WO 03/006602 and WO 04/099401.

Examples of useful variants are described in WO 92/19729, WO 98/20115, WO 98/20116, and WO 98/34946, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Examples of commercially available subtilisins include Kannase^{™}, Everlase^{™}, Primase^{™}, Duralase^{™}, Esperase^{™}, Alcalase^{™}, Durazym^{™}, Savinase^{™}, Ovozyme^{™}, Liquanase^{™}, Coronase^{™}, Polarzyme^{™}, Pyrase^{™}, and Clear-Lens^{™} Pro; Blaze^{™} (Novozymes A/S). Other commercially available proteases include Ronozyme^{™} Pro, Maxatase^{™}, Maxacal^{™}, Maxapem^{™}, Opticlean^{™}, Properase^{™}, Purafect^{™}, Purafect Ox^{™}, Purafact Prime^{™}, Excellase^{™}, FN2^{™}, FN3^{™} and FN4^{™} (available from Dupont).

In an embodiment of the invention, the subtilisin is subtilisin 309 or subtilisin BPN', or a variant of any of these. Preferably, the amino acid sequence of the subtilisin has at least 70% sequence identity, preferably at least 75%, more preferably at least 80%, more preferably at least 85%, more preferably at least 90%, more preferably at least 95%, 96%, 97%, 98%, 99%, and most preferably 100% sequence identity to the amino acid sequence of SEQ ID NO: 1 or SEQ ID NO: 2. In an embodiment, the amino acid sequence of the subtilisin is SEQ ID NO: 1 or SEQ ID NO: 2.

In an embodiment, the number of amino acid substitutions, deletions and/or insertions introduced into the amino acid sequence of SEQ ID NO: 1 or SEQ ID NO: 2 is up to 10, *e.g.,* 1, 2, 3, 4, 5, 6, 7, 8, 9, or 10; or up to 5, *e.g.,* 1, 2, 3, 4, or 5. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for subtilisin activity to identify amino acid residues that are critical to the activity of the molecule. See also, Hilton et al., 1996, J. Biol. Chem. 271: 4699-4708. The active site of the subtilisin or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et a/., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

The relatedness between two amino acid sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

### Non-subtilisin enzyme

The non-subtilisin enzyme to be combined with the subtilisin stabilizer (and the subtilisin), according to the invention, may be one or more non-subtilisin enzymes selected from the group consisting of lipase, cutinase, amylase, carbohydrase, cellulase, pectinase, pectate lyase, mannanase, arabinase, galactanase, xylanase, DNase, perhydrolase, and oxidoreductase (oxidase, laccase, peroxidase, haloperoxidase).

The methods and compositions of the invention may include 1, 2, 3, 4, 5, 6, 7, or 8 non-subtilisin enzyme(s). A non-subtilisin enzyme is an enzyme, preferably a detergent enzyme, which is not a subtilisin.

In general the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

### Lipase/cutinase

Suitable lipases and cutinases include those of bacterial or fungal origin. Chemically modified or protein engineered mutants are included. Examples include lipase from *Thermomyces, e.g.,* from *T. lanuginosus* (previously named *Humicola lanuginosa)* as described in EP 258068 and EP 305216, cutinase from *Humicola, e.g. H. insolens* as described in WO 96/13580, a *Pseudomonas* lipase, *e.g.,* from P. *alcaligenes* or P. *pseudoalcaligenes* (EP 218272), P. *cepacia* (EP 331376), *P. stutzeri* (GB 1372034), *P. fluorescens, Pseudomonas sp.* strain SD 705 (WO 95/06720 and WO 96/27002), P. *wisconsinensis* (WO 96/12012), a *Bacillus* lipase, *e.g.,* from B. *subtilis* (Dartois et al., Biochemica et Biophysica Acta, (1993), 1131, 253-360), *B. stearothermophilus* (JP 64/744992) or B. *pumilus* (WO 91/16422), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S*. *pristinaespiralis* (WO12/137147).

Other examples are lipase variants such as those described in WO 92/05249, WO 94/01541, EP 407225, EP 260105, WO 95/35381, WO 96/00292, WO 95/30744, WO 94/25578, WO 95/14783, WO 95/22615, WO 97/04079, WO 97/07202, WO 00/060063, WO 07/087508 and WO 09/109500.

Preferred commercially available lipase enzymes include Lipolase^{™}, Lipolase Ultra^{™}, and Lipex^{™}; Lecitase^{™}, Lipolex^{™}; Lipoclean^{™}, Lipoprime^{™} (Novozymes A/S). Other commercially available lipases include Lumafast (Genencor Int Inc); Lipomax (Gist-Brocades/Genencor Int Inc) and Bacillus sp lipase from Solvay.

### Carbohydrase

A carbohydrase is a general term for enzymes that cleave carbohydrates. In general carbohydrases are named after the substrates they act on, for example amylases act on amylase and cellulases act on cellulose. Many carbohydrases have found use in cleaning and laundry applications, such as amylase, cellulase, pectinase, pectate lyase, mannanase, arabinase, galactanase and xylanase, and all these can be applied in the liquid cleaning composition.

### Amylase

Suitable amylases include those of bacterial or fungal origin. Chemically modified or protein engineered mutants are included. Amylases include, for example, α-amylases obtained from *Bacillus, e.g.,* a special strain of *Bacillus licheniformis,* described in more detail in GB 1,296,839.

Examples of suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from B. *amyloliquefaciens* shown in SEQ ID NO: 6 of WO 2006/066594 and residues 36-483 of the B. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from B. *amyloliquefaciens* shown in SEQ ID NO: 6 of WO 2006/066594 and residues 36-483 of SEQ ID NO: 4 are those having the substitutions: M197T;
H156Y+A181T+N190F+A209V+Q264S; or
G48A+T49I+G107A+H156Y+A181T+N190F+1201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particularly preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Stainzyme^{™}, Stainzyme Plus^{™}, Amplify^{™}, Resilience^{™}, Everest^{™}, Duramyl^{™}, Termamyl^{™}, Termamyl Ultra^{™}; Natalase^{™}, Fungamyl^{™} and BAN^{™} (Novozymes A/S), Rapidase^{™} and Purastar^{™}/Effectenz^{™}, Powerase^{™} and Preferenz S100 (from Genencor International Inc./DuPont).

### Lyases

The lyase may be a pectate lyase derived from *Bacillus,* particularly B. *licherniformis* or B. *agaradhaerens,* or a variant derived of any of these, e.g. as described in US 6124127, WO 99/027083, WO 99/027084, WO 02/006442, WO 02/092741, WO 03/095638, Commercially available pectate lyases are XPect^{™}, Pectawash^{™}, and Pectaway^{™} (Novozymes A/S).

### Mannanase

The mannanase may be an alkaline mannanase of Family 5 or 26. It belongs It may be a wild-type from *Bacillus* or *Humicola,* particularly B. *agaradhaerens, B. licheniformis, B. halodurans, B. clausii,* or *H. insolens.* Suitable mannanases are described in WO 99/064619. A commercially available mannanase is Mannaway^{™} (Novozymes A/S).

### Cellulase

Suitable cellulases may be of bacterial or fungal origin. Chemically or genetically modified mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US

Commercially available cellulases include Carezyme^{™}, Celluzyme^{™}, Celluclean^{™}, Celluclast^{™}, Endolase^{™}, Renozyme^{™}, Whitezyme^{™} (Novozymes A/S); Clazinase^{™}, Puradax, Puradax HA, and Puradax EG (available from Genencor) and KAC-500(B)^{™} (Kao Corporation).

### Peroxidases/Oxidases

Suitable peroxidases are comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from C. *cinerea* (EP 179,486), and variants thereof as those described in WO 93/24618, WO 95/10602, and WO 98/15257.

The peroxidases also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g.,* C. *fumago, Alternaria, Curvularia, e.g., C. verruculosa* and C. *inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as C. *inaequalis* CBS 102.42 as described in WO 95/27046; or C. *verruculosa* CBS 147.63 or C. *verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

Suitable oxidases include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus,*

*Trametes, e.g., T. villosa* and *T. versicolor, Rhizoctonia, e.g., R. solani, Coprinopsis, e.g.,* C. *cinerea, C. comatus, C. friesii,* and C. *plicatilis, Psathyrella, e.g., P. condelleana, Panaeolus, e.g., P. papilionaceus, Myceliophthora, e.g., M. thermophila, Schytalidium, e.g., S.*

*thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.* A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

### Deoxyribonuclease (DNase)

Suitable deoxyribonucleases (DNases) are any enzyme that catalyzes the hydrolytic cleavage of phosphodiester linkages in the DNA backbone, thus degrading DNA. According to the invention, a DNase which is obtainable from a bacterium is preferred; in particular a DNase which is obtainable from a *Bacillus* is preferred; in particular a DNase which is obtainable from *Bacillus subtilis* or *Bacillus licheniformis* is preferred. Examples of such DNases are described in patent application WO 2011/098579 or in PCT/EP2013/075922.

### Perhydrolase

Suitable perhydrolases are capable of catalyzing a perhydrolysis reaction that results in the production of a peracid from a carboxylic acid ester (acyl) substrate in the presence of a source of peroxygen (e.g., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring *Mycobacterium* perhydrolase enzymes, or variants thereof. An exemplary enzyme is derived from *Mycobacterium smegmatis.* Such enzyme, its enzymatic properties, its structure, and variants thereof, are described in WO 2005/056782, WO 2008/063400, US 2008/145353, and US2007167344.

### Peptide aldehyde protease stabilizer

The peptide-derived protease stabilizer ("peptide protease stabilizer") used in the methods and compositions of the invention is a peptide aldehyde or a hydrosulfite adduct thereof.

The peptide aldehyde protease stabilizer has the formula P-B²-B¹-B⁰-H or an adduct having the formula P-B²-B¹-N(H)-CHR-CHOH-SO₃M, wherein
i) H is hydrogen;
ii) B⁰ is a single amino acid residue with L- or D-configuration of the formula -NH-CH(R)-C(=O)-;
iii) B¹ and B² are independently single amino acid residues;
iv) R is independently selected from the group consisting of C₁₋₆ alkyl, C₆₋₁₀ aryl or C₇₋₁₀ arylalkyl optionally substituted with one or more, identical or different, substituents R';
v) R' is independently selected from the group consisting of halogen, -OH, -OR", -SH, -SR", - NH₂, -NHR", -NR"₂, -CO₂H, -CONH₂, -CONHR", -CONR"₂, -NHC(=N)NH₂;
vi) R" is a C₁₋₆ alkyl group;
vii) P is an N-terminal protection group, preferably methoxycarbonyl (Moc) or benzyloxycarbonyl (Cbz); and
viii) M is H or an alkali metal, preferably Na or K.

B⁰ may be a single amino acid residue with L- or D-configuration, which is connected to H via the C-terminal of the amino acid. B⁰ has the formula -NH-CH(R)-C(=O)-, wherein R is a C₁₋₆ alkyl, C₆₋₁₀ aryl or C₇₋₁₀ arylalkyl side chain, such as methyl, ethyl, propyl, isopropyl, butyl, isobutyl, phenyl or benzyl, and wherein R may be optionally substituted with one or more, identical or different, substituent's R'. Particular examples of B⁰ are the D- or L-form of arginine (Arg), 3,4-dihydroxyphenylalanine, isoleucine (Ile), leucine (Leu), methionine (Met), norleucine (Nle), norvaline (Nva), phenylalanine (Phe), m-tyrosine, p-tyrosine (Tyr) and valine (Val). A particular embodiment is when B⁰ is leucine, methionine, phenylalanine, p-tyrosine and valine.

B¹, which is connected to B⁰ via the C-terminal of the amino acid, may be an aliphatic, hydrophobic and/or neutral amino acid. Examples of B¹ are alanine (Ala), cysteine (Cys), glycine (Gly), isoleucine (Ile), leucine (Leu), norleucine (Nle), norvaline (Nva), proline (Pro), serine (Ser), threonine (Thr) and valine (Val). Particular examples of B¹ are alanine, glycine, isoleucine, leucine and valine. A particular embodiment is when B¹ is alanine, glycine or valine.

B², which is connected to B¹ via the C-terminal of the amino acid, may be an aliphatic, hydrophobic, neutral and/or polar amino acid. Examples of B² are alanine (Ala), arginine (Arg), capreomycidine (Cpd), cysteine (Cys), glycine (Gly), isoleucine (Ile), leucine (Leu), norleucine (Nle), norvaline (Nva), phenylalanine (Phe), proline (Pro), serine (Ser), threonine (Thr), and valine (Val). Particular examples of B² are alanine, arginine, capreomycidine, glycine, isoleucine, leucine, phenylalanine and valine. A particular embodiment is when B² is arginine, glycine, leucine, phenylalanine or valine.

The N-terminal protection group P may be selected from formyl, acetyl (Ac), benzoyl (Bz), trifluoroacetyl, methoxysuccinyl, aromatic and aliphatic urethane protecting groups such as fluorenylmethyloxycarbonyl (Fmoc), methoxycarbonyl (Moc), (fluoromethoxy)carbonyl, benzyloxycarbonyl (Cbz), t-butyloxycarbonyl (Boc) and adamantyloxycarbonyl; p-methoxybenzyl carbonyl, benzyl (Bn), p-methoxybenzyl (PMB), p-methoxyphenyl (PMP), methoxyacetyl, methylamino carbonyl, methylsulfonyl, ethylsulfonyl, benzylsulfonyl, methylphosphoramidyl (MeOP(OH)(=O)) and benzylphosphoramidyl (PhCH₂OP(OH)(=O)).

Suitable peptide aldehydes are described in WO 2009/118375. More particularly, the peptide aldehyde may be Cbz-Arg-Ala-Tyr-H, Ac-Gly-Ala-Tyr-H, Cbz-Gly-Ala-Tyr-H, Cbz-Gly-Ala-Tyr-CF₃, Cbz-Gly-Ala-Leu-H, Cbz-Val-Ala-Leu-H, Cbz-Val-Ala-Leu-CF₃, Moc-Val-Ala-Leu-CF₃, Cbz-Gly-Ala-Phe-H, Cbz-Gly-Ala-Phe-CF₃, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, MeO-CO-Val-Ala-Leu-H, MeNCO-Val-Ala-Leu-H, MeSO₂-Val-Ala-Leu-H, PhCH₂O-P(OH)(O)-Val-Ala-Leu-H, PhCH₂SO₂-Val-Ala-Leu-H, PhCH₂O-P(OH)(O)-Leu-Ala-Leu-H, or PhCH₂O-P(OH)(O)-Phe-Ala-Leu-H, A preferred stabilizer for use in the liquid composition of the invention is Cbz-Gly-Ala-Tyr-H, or a hydrosulfite adduct thereof, wherein Cbz is benzyloxycarbonyl.

### Liquid detergent composition

The liquid detergent composition has a physical form, which is not solid (or gas). It may be a pourable liquid, a pourable gel or a non-pourable gel. It may be isotropic or structured, preferably isotropic. It may be a formulation useful for washing in automatic washing machines or for hand washing.

The liquid detergent composition may be aqueous, typically containing at least 20% by weight and up to 95% water, such as up to 70% water, up to 50% water, up to 40% water, up to 30% water, or up to 20% water. Other types of liquids, including without limitation, alkanols, amines, diols, ethers and polyols may be included in an aqueous liquid detergent. An aqueous liquid detergent may contain from 0-30% organic solvent. A liquid detergent may even be nonaqueous, wherein the water content is below 10%, preferably below 5%.

Detergent ingredients can be separated physically from each other by compartments in water dissolvable pouches. Thereby negative storage interaction between components can be avoided. Different dissolution profiles of each of the compartments can also give rise to delayed dissolution of selected components in the wash solution.

The detergent composition may take the form of a unit dose product. A unit dose product is the packaging of a single dose in a non-reusable container. It is increasingly used in detergents for laundry and dish wash. A detergent unit dose product is the packaging (e.g., in a pouch made from a water soluble film) of the amount of detergent used for a single wash.

Pouches can be of any form, shape and material which is suitable for holding the composition, e.g., without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be a blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticizers like glycerol, ethylene glycerol, Propylene glycol, sorbitol and mixtures thereof. The pouches can comprise a solid cleaning composition or part components and/or a liquid cleaning composition or part components separated by the water soluble film. The compartment for liquid components can be different in composition than compartments containing solids (see *e*.*g*., US 2009/0011970).

The choice of detergent components may include the consideration of the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

The choice of additional components is within the skill of the artisan and includes conventional ingredients, including the exemplary non-limiting components set forth below.

### Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or *N*-acyl *N*-alkyl derivatives of glucosamine (glucamides, GA, or fatty acid glucamide, FAGA), as well as products available under the trade names SPAN and TWEEN, and combinations thereof.

When included therein the detergent will usually contain from about 0.1% to about 20% by weight of a semipolar surfactant. Non-limiting examples of semipolar surfactants include amine oxides (AO) such as alkyldimethylamineoxide, N-(coco alkyl)-*N*,*N*-dimethylamine oxide and N-(tallow-alkyl)-*N*,*N*-bis(2-hydroxyethyl)amine oxide, fatty acid alkanolamides and ethoxylated fatty acid alkanolamides, and combinations thereof.

When included therein the detergent will usually contain from about 0.1% to about 10% by weight of a zwitterionic surfactant. Non-limiting examples of zwitterionic surfactants include betaine, alkyldimethylbetaine, sulfobetaine, and combinations thereof.

### Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg ions. Any builder and/or co-builder known in the art for use in laundry detergents may be utilized. Non-limiting examples of builders include citrates, zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 0.5% to about 10%, of a detergent co-builder, or a mixture thereof. The detergent composition may include include a co-builder alone, or in combination with a builder, for example a citrate builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), N-(2-sulfomethyl)-glutamic acid (SMGL), *N*-(2-sulfoethyl)-glutamic acid (SEGL), N-methyliminodiacetic acid (MIDA), α-alanine-*N*, *N*-diacetic acid (a-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA) , taurine-*N*, *N*-diacetic acid (TUDA) and sulfomethyl-*N*, *N*-diacetic acid (SMDA), *N*-(2-hydroxyethyl)-ethylidenediamine-*N*, *N*', *N*'-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e*.*g*., WO 09/102854, US 5977053.

### Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, *e*.*g*., WO 2006/130575 and US 5,955,415. Salts of the above-mentioned polymers are also contemplated.

### Additional enzymes

The liquid detergent composition may comprise additional enzymes using other formulation technologies, such as encapsulation (e.g., as described in WO 1997/024177, WO 2014/177709 or WO 2015/144784), (matrix) particles, or enzymatic water soluble films (e.g., as described in WO 2014/152674, WO 2014/152547, or WO 2013/148492).

### Adjunct materials

Any detergent components known in the art for use in laundry detergents may also be utilized.

### Bleaching Systems

Due to the incompatibility of the components there are still only few examples of liquid detergents combining bleach and enzymes (e.g., US 5,275,753 or WO 99/00478). The detergent may contain 0-50% of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae: 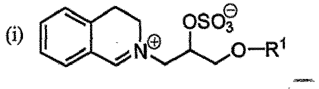 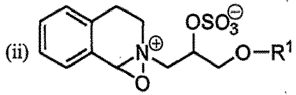 and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e*.*g*., in WO 2007/087258, WO 2007/087244, WO 2007/087259 and WO 2007/087242. Suitable photobleaches may for example be sulfonated zinc phthalocyanine.

### Formulation of detergent products

The liquid detergent composition of the invention may be in any convenient form, *e*.*g*., a pouch having one or more compartments, a gel, or a regular, compact or concentrated liquid detergent (see *e.g*., WO 2009/098660 or WO 2010/141301).

Pouches can be configured as single or multi compartments. It can be of any form, shape and material which is suitable for holding the composition, *e*.*g*., without allowing release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid cleaning composition or part components and/or a liquid cleaning composition or part components separated by the water soluble film. The compartment for liquid components can be different in composition than compartments containing solids.

Detergent ingredients can be separated physically from each other by compartments in water dissolvable pouches. Thereby negative storage interaction between components can be avoided. Different dissolution profiles of each of the compartments can also give rise to delayed dissolution of selected components in the wash solution.

### EXAMPLES

Chemicals used as buffers and substrates were commercial products of at least reagent grade. Protease1 has the amino acid sequence as shown in SEQ ID NO: 1. Protease2 has the amino acid sequence as shown in SEQ ID NO: 3. Protease3 has the amino acid sequence as shown in SEQ ID NO: 4. The protease stabilizer used was Cbz-Gly-Ala-NHCH(CH₂Cr,H₄pOH)C(OH)(SO₃)Na, wherein Cbz is benzyloxycarbonyl (hydrosulfite/bisulfite form of a peptide aldehyde).

Carbonate buffer was prepared by mixing 5 g of sodium bicarbonate and 1 g of sodium carbonate in 1 L distilled water, and adjusting to pH 9.

The experiments for this patent application were performed with the standard test kits described below.

These test kits are originally developed for Hospital uses (to ensure and verify the good performances of the washer disinfectors and cleaning solutions on a regular basis) and qualification companies who have to certify the good efficacy of the medical instruments reprocessing equipment's/materials installed in medical center (dentist, hospital...).

The test kits used in this patent application are all compliant with ISO 15883 related to the washer disinfectors.

### Test kits

### SIMICON-RI cleaning indicator

Blood stain for washer disinfector (Fibrin + Hemoglobin protein); from SIMICON GmbH, Sigmund-Riefler-Bogen 19, 81829 München - Riem, Germany.

*Field of application:* The cleaning indicator SIMICON RI is designed for the validation and the routine monitoring of the cleaning efficacy of cleaning and disinfection process in the washer desinfectors for standard and minimally invasive surgery (MIS) instruments.

*Features:* The cleaning indicator SIMICON RI is contaminated with test soil according to ISO 15883.

*Conformity:* The resistance of the cleaning indicator SIMICON RI is adjusted in accordance with ISO 15883. If the essential parameters such as time, temperature, water pressure and detergent concentration are accurately calibrated the spot of test will be removed completely at the end of the cleaning cycle.

### Specifications:

- Test soil: according to ISO 15883
- Carrier: Stainless steel V4A
- Organic burden: sheep blood and additives

### 'STF load check' cleaning indicator

Multi direction indicator with 4 faces per sheet and various types of Protein/Lipid/Polysaccharide soil; from Albert BROWNE Ltd, Chancery House, 190 Waterside Road, Hamilton Industrial Park, Leicester LE5 1QZ, UK.

*Field of application:* The 'STF load check' indicator test is a consistent, repeatable way to monitor the cleaning efficacy of your washer disinfector. The indicator strips have been designed to perform in an equivalent manner to the test soils described in documents such as ISO 15883-5, HTM2030 and HTM01-05. When placed in the 'STF Load Check' holders, they represent typical soiling that would occur during normal use of reusable surgical instruments. This is a bench marked performance test which is suitable for validation. The indicator strips mimic occluded surfaces to present a realistic washing cleaning challenge.

*Features:* The 'STF load check' indicator strips contain two sources of protein, lipids and polysaccharides.

*Conformity:* The resistance of the cleaning indicator 'STF load check' is adjusted in accordance with ISO 15883, HTM2030 and HTM01-05. If the essential parameters such as time, temperature, water pressure and detergent concentration are accurately calibrated the spot of test will be removed completely at the end of the cleaning cycle.

### Specifications:

- Test soil: according to ISO 15883-5, HTM2030 and HTM01-05
- Carrier: plastic sheets
- Organic burden: two sources of protein, lipids and polysaccharides.

### TOSI cleaning indicator

Blood soil on stainless steel coupon. Mixture of different sources of proteins (hemoglobin and fibrin). Available from Healthmark Industries Company, 33671 Doreka, Fraser, MI 48026, United States.

*Field of application:* TOSI is a blood soil device that directly correlates to the cleaning challenge of surgical instruments. TOSI device provides a consistent, repeatable, and reliable method for evaluating the cleaning effectiveness of the automated instrument washer. The blood soil is manufactured to exacting specifications each and every time. When metered on to the stainless steel plate, the TOSI is completely analogous to a stainless steel instrument soiled with dried blood. Placed in the see-through plastic holder, the challenge is identical to the areas of instruments typically hidden from view (i.e., box locks). The routine use of this test will help insure that your instrument washer is performing at a consistent level, enhancing the routine visual inspection of instruments.

*Features:* The TOSI indicator contains several sources of protein: hemoglobin, albumin and fibrin.

*Conformity:* The resistance of the cleaning indicator TOSI is adjusted in accordance with ISO 15883, AAMI and AORN Guidelines as well as ASTM Guide D7225.

### Specifications:

- Test soil: according to ISO 15883-5, AAMI and AORN Guidelines and ASTM Guide D7225
- Carrier: stainless steel
- Organic burden: several sources of protein - fibrin, albumin and hemoglobin.

### EXAMPLE 1

### Washing performance measured with SIMICON-RI cleaning indicator

1. A liquid enzymatic solution was made by preparing a carbonate buffer at pH 9, adding 1% w/w surfactant (70% mergital D8 and 30% Dehydol PO5), and adding 0.1% w/w enzyme. The solution was transferred to a 200 ml beaker (big enough to enable immersion of the test kit).

The carbonate buffer was pre-heated at 40°C in a heating bath before adding the enzymes and surfactant, and both were added just before the wash test.

2. A magnet stirrer was added to the wash solution. The beaker was placed on a heating plate where temperature and mechanical action (stirring system) was controlled. The temperature was set at 40°C and the stirring action at 150 rpm.

3. The SIMICON RI medical cleaning indicator was taken out of the package and placed in a holder with a plier. The cleaning indicator was immersed at the center of the beaker, in the vortex generated by the magnet stirrer. Always put the indicator at the same area for the whole test series. Changing this area in the beaker can significantly modify the washing performance.

4. The cleaning performance was determined by measuring the time required to reach 100% soil removal on the surface of the indicator (visual observation). An average of several replicates was calculated to increase accuracy of the results.

5. The shorter the time to obtain 100% soil removal, the better and more efficient the enzyme tested is (see Table 1). Table 1. **SIMICON RI;** Time required to reach 100% soil removal (minutes).

| **Conditions** | **Protease** | **Time** (minutes) |
|---|---|---|
| 1% surfactant; pH 9; 40°C; Stirring; 0.1% protease | Protease 1 | 25.6 |
| | Protease1 + protease stabilizer | 20.2 |
| | Protease2 | 16.3 |
| | Protease2 + protease stabilizer | 14.4 |

### EXAMPLE 2

### Washing performance measured with STF load check cleaning indicator (Protocol 1)

1. A liquid enzymatic solution without surfactant was made by preparing a carbonate buffer at pH 9 and adding 0.02% w/w enzyme. The solution was transferred to a 200 ml beaker (big enough to enable immersion of the test kit).

The carbonate buffer was pre-heated at 40°C in a heating bath before adding the enzymes, just before the wash test.

2. A magnet stirrer was added to the wash solution. The beaker was placed on a heating plate where temperature and mechanical action (stirring system) was controlled. The temperature was set at 40°C and the stirring action at 150 rpm.

3. The 'STF load check' medical cleaning indicator was taken out of the package and placed in a holder with a plier. The cleaning indicator was immersed at the center of the beaker, in the vortex generated by the magnet stirrer. Always put the indicator at the same area for the whole test series. Changing this area in the beaker can significantly modify the washing performance.

4. The cleaning performance was determined by measuring the time required to reach 100% soil removal on the surface of the indicator (visual observation). An average of several replicates was calculated to increase accuracy of the results.

5. The shorter the time to obtain 100% soil removal, the better and more efficient the enzyme tested is (see Table 2). Table 2. **STF load check;** Time required to reach 100% soil removal (minutes).

| **Conditions** | **Protease** | **Time** (minutes) |
|---|---|---|
| No surfactant; pH 9; 40°C; Stirring; 0.02% protease | Protease 1 | 8.4 |
| | Protease1 + protease stabilizer | 7.8 |
| | No enzyme | >30 |

### EXAMPLE 3

### Washing performance measured with STF load check cleaning indicator (Protocol 2)

Protocol 1 (see above) was followed from step 1 to step 5.
6. The 'STF load check' cleaning indicator was removed after 4 minutes of washing in the beaker
7. The cleaning indicator was rinsed in tap water for 5 seconds without touching the remaining soiled part.
8. The cleaning indicator was dried while being careful not to touch the indicator surface against towel/tissue or anything else to not remove remaining soil from the surface.
9. The cleaning performance was determined by measuring the reflectance value at 460 nm with DIGleye equipment of the 'STF load check'. An average of several replicates was calculated to increase accuracy of the results.
10. The higher the reflectance value, the better and more efficient was the enzyme (see Table 3). Table 3. **STF load check;** Reflectance measured at 460 nm after stirring for 4 minutes.

| **Conditions** | **Protease** | **Reflectance** |
|---|---|---|
| No surfactant; | Protease1 | 25.8 |

| | | |
|---|---|---|
| pH 9; 40°C; Stirring; 0.02% protease | Protease1 + protease stabilizer | 70.0 |

### EXAMPLE 4

### Soaking without stirring measured with STF load check cleaning indicator (Protocol 2)

Protocol 2 was performed without stirring. In some cases, medical instrument reprocessing is done by soaking only. Mechanical action can be manual cleaning or during the automatic cleaning (after the manual cleaning).

The 'STF load check' indicator was placed in a beaker without letting any side of the indicator in contact with the surface of the beaker. The only difference in this procedure compared to the "stirring" protocol was the time required: the 'STF load check' indicator was immersed in the enzymatic soaking solution for 18 minutes before removing the indicator and rinsing it.

The higher the reflectance value, the better and more efficient was the enzyme tested (see Table 4).

**Table 4. STF load check; Reflectance measured at 460 nm after soaking for 18 minutes.**

| **Conditions** | **Protease** | **Reflectance** |
|---|---|---|
| No surfactant; pH 9; 40°C; No stirring; 0.02% protease | Protease1 | 46.7 |
| | Protease1 + protease stabilizer | 54.7 |
| | Protease2 | 61.5 |
| | Protease2 + protease stabilizer | 62.3 |

### EXAMPLE 5

### Washing performance in commercial medical detergents; STF load check or TOSI cleaning indicator

1. A liquid enzymatic detergent/washing solution was prepared in a beaker and pre-heated at 40°C in a heating bath. The enzyme concentration in the wash solution was 0.0125%. The detergent was either a commercial European medical detergent (Table 5) or a commercial Asia Pacific medical detergent (Table 6 and Table 7). The pH was determined by the medical detergent used.
2. A magnet stirrer was added to the wash solution (total wash solution was 200 ml). The beaker was placed on a heating plate where temperature and mechanical action (stirring system) was controlled. The temperature was set at 40°C and the stirring action at 150 rpm.
3. The 'STF load check' medical cleaning indicator or the TOSI medical cleaning indicator was taken out of the package and placed in a holder with a plier. The cleaning indicator was immersed at the center of the beaker, in the vortex generated by the magnet stirrer. Always put the indicator at the same area for the whole test series. Changing this area in the beaker can significantly modify the washing performance.
4. The cleaning performance was determined by measuring the time required to reach 100% soil removal on the surface of the indicator (visual observation). An average of two replicates was calculated to increase accuracy of the results.
5. The shorter the time to obtain 100% soil removal, the better and more efficient the enzyme is (see Tables 5-7). Table 5. **STF load check;** Time required to reach 100% soil removal (minutes).

| **Conditions** | **Protease** | **Time** (minutes) |
|---|---|---|
| European medical detergent; pH 9.5; 40°C; Stirring; 0.0125% protease | Protease 1 | 22.3 |
| | Protease1 + protease stabilizer | 13.6 |
| | Protease2 + protease stabilizer | 9.2 |
| | Protease3 + protease stabilizer | 6.2 |

Table 6. **STF load check;** Time required to reach 100% soil removal (minutes).

| **Conditions** | **Protease** | **Time** (minutes) |
|---|---|---|
| Asia Pacific medical detergent; pH 8.9; 40°C; Stirring; 0.0125% protease | Protease1 | 36.5 |
| | Protease1 + protease stabilizer | 24.0 |
| | Protease2 + protease stabilizer | 15.4 |
| | Protease3 + protease stabilizer | 8.2 |

Table 7. **TOSI;** Time required to reach 100% soil removal (minutes).

| **Conditions** | **Protease** | **Time** (minutes) |
|---|---|---|
| Asia Pacific medical detergent; pH 8.9; 40°C; Stirring; 0.0125% protease | Protease1 | 18.0 |
| | Protease1 + protease stabilizer | 16.2 |
| | Protease2 + protease stabilizer | 10.9 |
| | Protease3 + protease stabilizer | 8.2 |

## Claims

1. A method for cleaning a medical or dental instrument comprising:
(a) soaking the medical or dental instrument in an aqueous composition comprising
i) from 0.001% w/w to 1% w/w of a protease, wherein the protease is subtilisin, and
ii) from 0.001% w/w to 1% w/w of a peptide aldehyde protease stabilizer;
(b) optionally washing the instrument with a liquid detergent composition; and
(c) rinsing the instrument;
wherein the peptide aldehyde protease stabilizer has the formula P-B²-B¹-B⁰-H or a hydrosulfite adduct thereof having the formula P-B²-B¹-N(H)-CHR-CHOH-SO₃M, wherein
i) H is hydrogen;
ii) B⁰ is a single amino acid residue with L- or D-configuration of the formula -NH-CH(R)-C(=O)-;
iii) B¹ and B² are independently single amino acid residues;
iv) R is independently selected from the group consisting of C₁₋₆ alkyl, C₆₋₁₀ aryl or C₇₋₁₀ arylalkyl optionally substituted with one or more, identical or different, substituents R';
v) R' is independently selected from the group consisting of halogen, -OH, -OR", -SH, -SR", - NH₂, -NHR", -NR"₂, -CO₂H, -CONH₂, -CONHR", -CONR"₂, -NHC(=N)NH₂;
vi) R" is a C₁₋₆ alkyl group;
vii) P is an N-terminal protection group; and
viii) M is H or an alkali metal, preferably Na or K.

2. The method of claim 1, wherein the medical or dental instrument is soaked in the aqueous composition in (a) for a time sufficient to reduce soil on the instrument, preferably for at least 1 minute.

3. The method of any one of claims 1-2, wherein the detergent composition in (b) consists of a surfactant and other ingredients used in detergent and cleaning compositions.

4. The method of any one of claims 1-3, wherein the aqueous composition in (a) further comprises one or more enzymes selected from the group consisting of amylase, lipase, cellulase, pectinase, mannanase, DNase, perhydrolase, and oxidoreductase.

5. The method of any one of claims 1-4, wherein the aqueous composition in (a) further contains a surfactant or a wetting agent.

6. The method of any one of claims 1-5, wherein the aqueous composition in (a) has an alkaline pH, preferably a pH in the range of pH 7 to pH 10.

7. The method of any one of claims 1-6, wherein B⁰ is leucine, methionine, phenylalanine, p-tyrosine, or valine; preferably leucine, phenylalanine, or p-tyrosine.

8. The method of any one of claims 1-7, wherein B¹ is alanine, glycine, or valine.

9. The method of any one of claims 1-8, wherein B² is arginine, glycine, leucine, phenylalanine, or valine; preferably arginine, glycine, or valine.

10. The method of any one of claims 1-9, wherein P is p-methoxycarbonyl (Moc) or benzyloxycarbonyl (Cbz).

11. The method of any one of claims 1-10, wherein the peptide aldehyde protease stabilizer is Cbz-Arg-Ala-Tyr-H, Ac-Gly-Ala-Tyr-H, Cbz-Gly-Ala-Tyr-H, Cbz-Gly-Ala-Tyr-CF₃, Cbz-Gly-Ala-Leu-H, Cbz-Val-Ala-Leu-H, Cbz-Val-Ala-Leu-CF₃, Moc-Val-Ala-Leu-CF₃, Cbz-Gly-Ala-Phe-H, Cbz-Gly-Ala-Phe-CF₃, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, MeO-CO-Val-Ala-Leu-H, MeNCO-Val-Ala-Leu-H, MeSO₂-Val-Ala-Leu-H, PhCH₂O-P(OH)(O)-Val-Ala-Leu-H, PhCH₂SO₂-Val-Ala-Leu-H, PhCH₂O-P(OH)(O)-Leu-Ala-Leu-H, or PhCH₂O-P(OH)(O)-Phe-Ala-Leu-H, or a hydrosulfite adduct of any of these, wherein Cbz is benzyloxycarbonyl and Moc is methoxycarbonyl.

12. The method of any one of claims 1-11, wherein the stabilizer is Cbz-Gly-Ala-Tyr-H or Moc-Val-Ala-Leu-H, or a hydrosulfite adduct thereof, wherein Cbz is benzyloxycarbonyl and Moc is methoxycarbonyl.

13. The method of any one of claims 1-12, wherein cleaning means reducing the amount of blood, blood constituents, blood proteins, fibrin, albumin, and/or hemoglobin.

## Patentansprüche

1. Verfahren zum Reinigen eines medizinischen oder zahnmedizinischen Instruments, umfassend:
(a) Einweichen des medizinischen oder zahnmedizinischen Instruments in einer wässrigen Zusammensetzung, umfassend
i) von 0,001% Gew./Gew. bis 1% Gew./Gew. einer Protease, wobei die Protease Subtilisin ist, und
ii) von 0,001% Gew./Gew. bis 1% Gew./Gew. eines Peptidaldehyd-Proteasestabilisators;
(b) gegebenenfalls Waschen des Instruments mit einer flüssigen Detergenszusammensetzung; und
(c) Spülen des Instruments;
wobei der Peptidaldehyd-Proteasestabilisator die Formel P-B²⁻B¹-B⁰-H oder ein Hydrosulfitaddukt davon mit der Formel P-B²-B¹-N(H)-CHR-CHOH-SO₃M aufweist, wobei
i) H Wasserstoff ist;
ii) B⁰ ein einzelner Aminosäurerest mit L- oder D-Konfiguration der Formel -NH-CH(R)-C(=O)- ist;
iii) B¹ und B² unabhängig einzelne Aminosäurereste sind;
iv) R unabhängig ausgewählt ist aus der Gruppe bestehend aus C₁₋₆-Alkyl, C₆₋₁₀-Aryl oder C₇₋₁₀-Arylalkyl, gegebenenfalls substituiert mit einem oder mehreren identischen oder verschiedenen Substituenten R';
v) R' unabhängig ausgewählt ist aus der Gruppe bestehend aus Halogen, -OH, -OR", -SH, -SR", -NH₂, -NHR", -NR"₂, -CO₂H, -CONH₂, -CONHR", -CONR"₂, -NHC(=N)NH₂;
vi) R" eine C₁₋₆-Alkylgruppe ist;
vii) P eine N-terminale Schutzgruppe ist; und
viii) M H oder ein Alkalimetall ist, bevorzugt Na oder K.

2. Verfahren nach Anspruch 1, wobei das medizinische oder zahnmedizinische Instrument in der wässrigen Zusammensetzung in (a) für eine Zeit eingeweicht wird, die ausreichend ist, eine Verschmutzung des Instruments zu reduzieren, bevorzugt für mindestens 1 Minute.

3. Verfahren nach einem beliebigen der Ansprüche 1 - 2, wobei die Detergenszusammensetzung in (b) aus einem oberflächenaktiven Mittel und anderen Bestandteilen besteht, die in Detergens- und Reinigungsmittelzusammensetzungen verwendet werden.

4. Verfahren nach einem beliebigen der Ansprüche 1 - 3, wobei die wässrige Zusammensetzung in (a) des Weiteren ein oder mehrere Enzyme enthält, die aus der Gruppe bestehend aus Amylase, Lipase, Cellulase, Pektinase, Mannanase, DNase, Perhydrolase und Oxidoreduktase ausgewählt sind.

5. Verfahren nach einem beliebigen der Ansprüche 1 - 4, wobei die wässrige Zusammensetzung in (a) des Weiteren ein oberflächenaktives Mittel oder ein Benetzungsmittel enthält.

6. Verfahren nach einem beliebigen der Ansprüche 1-5, wobei die wässrige Zusammensetzung in (a) einen alkalischen pH, bevorzugt einen pH im Bereich von pH 7 bis pH 10 aufweist.

7. Verfahren nach einem beliebigen der Ansprüche 1-6, wobei B⁰ Leucin, Methionin, Phenylalanin, p-Tyrosin oder Valin ist, bevorzugt Leucin, Phenylalanin oder p-Tyrosin.

8. Verfahren nach einem beliebigen der Ansprüche 1-7, wobei B¹ Alanin, Glycin oder Valin ist.

9. Verfahren nach einem beliebigen der Ansprüche 1-8, wobei B² Arginin, Glycin, Leucin, Phenylalanin oder Valin ist, bevorzugt Arginin, Glycin oder Valin.

10. Verfahren nach einem beliebigen der Ansprüche 1-9, wobei P p-Methoxycarbonyl (Moc) oder Benzyloxycarbonyl (Cbz) ist.

11. Verfahren nach einem beliebigen der Ansprüche 1-10, wobei der Peptidaldehyd-Proteasestabilisator Cbz-Arg-Ala-Tyr-H, Ac-Gly-Ala-Tyr-H, Cbz-Gly-Ala-Tyr-H, Cbz-Gly-Ala-Tyr-CF₃, Cbz-Gly-Ala-Leu-H, Cbz-Val-Ala-Leu-H, Cbz-Val-Ala-Leu-CF₃, Moc-Val-Ala-Leu-CF₃, Cbz-Gly-Ala-Phe-H, Cbz-Gly-Ala-Phe-CF₃, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, MeO-CO-Val-Ala-Leu-H, MeNCO-Val-Ala-Leu-H, MeSO₂-Val-Ala-Leu-H, PhCH₂O-P(OH)(O)-Val-Ala-Leu-H, PhCH₂SO₂-Val-Ala-Leu-H, PhCH₂O-P(OH)(O)-Leu-Ala-Leu-H, oder PhCH₂O-P(OH)(O)-Phe-Ala-Leu-H, oder ein Hydrosulfitaddukt von einem beliebigen dieser ist, wobei Cbz Benzyloxycarbonyl ist, und Moc Methoxycarbonyl ist.

12. Verfahren nach einem beliebigen der Ansprüche 1-11, wobei der Stabilisator Cbz-Gly-Ala-Tyr-H oder Moc-Val-Ala-Leu-H oder ein Hydrosulfitaddukt davon ist, wobei Cbz Benzyloxycarbonyl ist, und Moc Methoxycarbonyl ist.

13. Verfahren nach einem beliebigen der Ansprüche 1-12, wobei Reinigen ein Verringern der Menge an Blut, Blutbestandteilen, Blutproteinen, Fibrin, Albumin und/oder Hämoglobin bedeutet.

## Revendications

1. Méthode de nettoyage d'un instrument médical ou dentaire, comprenant :
(a) l'immersion de l'instrument médical ou dentaire dans une composition aqueuse comprenant
i) de 0,001 % p/p à 1 % p/p d'une protéase, où la protéase est la subtilisine, et
ii) de 0,001 % p/p à 1 % p/p d'un stabilisant de protéase d'aldéhyde peptidique ;
(b) éventuellement le lavage de l'instrument avec une composition détergente liquide ; et
(c) le rinçage de l'instrument ;
dans laquelle le stabilisant de protéase d'aldéhyde peptidique a pour formule P-B²-B¹-B⁰-H ou un produit d'addition d'hydrosulfite de celui-ci ayant pour formule P-B²-B¹-N(H)-CHR-CHOH-SO₃M, dans laquelle
i) H est hydrogène ;
ii) B⁰ est un unique résidu d'acide aminé ayant une configuration L ou D de formule -NH-CH(R)-C(=O)- ;
iii) B¹ et B² sont indépendamment des uniques résidus d'acides aminés ;
iv) R est indépendamment choisi dans le groupe consistant en alkyle en C₁₋₆, aryle en C₆₋₁₀ ou arylalkyle en C₇₋₁₀, éventuellement substitué par un ou plusieurs substituants R', identiques ou différents ;
v) R' est choisi indépendamment dans le groupe consistant en halogène, -OH, -OR", -SH, -SR", -NH₂, -NHR", -NR"₂, -CO₂H, -CONH₂, -CONHR", -CONR"₂, -NHC(=N)NH₂ ;
vi) R" est un groupe alkyle en C₁₋₆ ;
vii) P est un groupe de protection N-terminal ; et
viii) M est H ou un métal alcalino-terreux, de préférence Na ou K.

2. Méthode selon la revendication 1, dans laquelle l'instrument médical ou dentaire est immergé dans la composition aqueuse à l'étape (a) pendant une durée suffisante pour réduire la souillure sur l'instrument, de préférence pendant au moins 1 minute.

3. Méthode selon l'une quelconque des revendications 1 à 2, dans laquelle la composition détergente à l'étape (b) consiste en un tensioactif et d'autres ingrédients utilisés dans les compositions détergentes et nettoyantes.

4. Méthode selon l'une quelconque des revendications 1 à 3, dans laquelle la composition aqueuse à l'étape (a) comprend en outre une ou plusieurs enzymes choisies dans le groupe consistant en amylase, lipase, cellulase, pectinase, mannanase, DNase, perhydrolase, et oxydoréductase.

5. Méthode selon l'une quelconque des revendications 1 à 4, dans laquelle la composition aqueuse à l'étape (a) comprend en outre un tensioactif ou un agent mouillant.

6. Méthode selon l'une quelconque des revendications 1 à 5, dans laquelle la composition aqueuse à l'étape (a) a un pH alcalin, de préférence un pH dans la plage de pH 7 à pH 10.

7. Méthode selon l'une quelconque des revendications 1 à 6, dans laquelle B⁰ est la leucine, la méthionine, la phénylalanine, la *p-*tyrosine, ou la valine ; de préférence la leucine, la phénylalanine ou la *p*-tyrosine.

8. Méthode selon l'une quelconque des revendications 1 à 7, dans laquelle B¹ est l'alanine, la glycine ou la valine.

9. Méthode selon l'une quelconque des revendications 1 à 8, dans laquelle B² est l'arginine, la glycine, la leucine, la phénylalanine ou la valine ; de préférence l'arginine, la glycine, ou la valine.

10. Méthode selon l'une quelconque des revendications 1 à 9, dans laquelle P est le p-méthoxycarbonyle (Moc) ou le benzyloxycarbonyle (Cbz).

11. Méthode selon l'une quelconque des revendications 1 à 10, dans laquelle le stabilisant de protéase d'aldéhyde peptidique est Cbz-Arg-Ala-Tyr-H, Ac-Gly-Ala-Tyr-H, Cbz-Gly-Ala-Tyr-H, Cbz-Gly-Ala-Tyr-CF₃, Cbz-Gly-Ala-Leu-H, Cbz-Val-Ala-Leu-H, Cbz-Val-Ala-Leu-CF₃, Moc-Val-Ala-Leu-CF₃, Cbz-Gly-Ala-Phe-H, Cbz-Gly-Ala-Phe-CF₃, Cbz-Gly-Ala-Val-H, Cbz-Gly-Gly-Tyr-H, Cbz-Gly-Gly-Phe-H, Cbz-Arg-Val-Tyr-H, Cbz-Leu-Val-Tyr-H, MeO-CO-Val-Ala-Leu-H, MeNCO-Val-Ala-Leu-H, MeSO₂-Val-Ala-Leu-H, PhCH₂O-P(OH)(O)-Val-Ala-Leu-H, PhCH₂SO₂-Val-Ala-Leu-H, PhCH₂O-P(OH)(O)-Leu-Ala-Leu-H, ou PhCH₂O-P(OH)O-Phe-Ala-Leu-H, ou un produit d'addition d'hydrosulfite de l'un quelconque de ceux-ci, où Cbz est le benzyloxycarbonyle et Moc est le méthoxycarbonyle.

12. Méthode selon l'une quelconque des revendications 1 à 11, dans laquelle le stabilisant est Cbz-Gly-Ala-Tyr-H ou Moc-Val-Ala-Leu-H, ou un produit d'addition d'hydrosulfite de l'un quelconque de ceux-ci, où Cbz est le benzyloxycarbonyle et Moc est le méthoxycarbonyle.

13. Méthode selon l'une quelconque des revendications 1 à 12, dans laquelle les moyens de nettoyage réduisent la quantité de sang, de constituants sanguins, de protéines sanguines, de fibrine, d'albumine, et/ou d'hémoglobine.
